# EUROPEAN PATENT APPLICATION

(11) **EP 4 840 394 A1**
(43) Date of publication of application: **18.11.2026**
(21) Application number: 25226293.6
(22) Date of filing: 22.12.2025
(51) Int. Cl.: A61B 5/0205, A61B 5/024, A61B 5/103, A61B 5/11, A61B 8/00

(54) **HEALTH ANALYSIS SYSTEM**

(30) Priority: 16.05.2025 TW 114118537
(71) Applicant: Tai, Hsu-Chih, Tainan City 702007 (TW)
(72) Inventor: Tai, Hsu-Chih, Tainan City 702007 (TW)
(74) Representative: Viering, Jentschura & Partner mbB Patent- und Rechtsanwälte

(57) **Abstract**

A health analysis system (1) includes a camera (10), a posture recognition module (40), a posture analysis module (50) and a health analysis module (60). The camera (10) is used for capturing an user image of the user. The posture recognition module (40) is electrically connected to the camera (10) and is used for recognizing a plurality of human skeleton points (A, A1, A2, A3, A4, A5, A6) in the user's image. The posture analysis module (50) is electrically connected to the posture recognition module (40) and is used for analyzing a human posture according to the plurality of human skeleton points (A, A1, A2, A3, A4, A5, A6). The health analysis module (60) is electrically connected to the posture analysis module (50), and further includes a skeleton analysis unit (61) which is used for analyzing the health status according to the human posture.

## Description

### BACKGROUND OF THE INVENTION

### 1. Field of the Invention

The present invention relates to a health analysis system, particularly to a health analysis system that analyzes health status according to human posture.

### 2. Description of the Related Art

As Taiwan enters an aging society, the health issues of the elderly gradually place significant pressure on social economics, medical, and welfare systems. As the elderly age, they may gradually develop common diseases of the elderly, such as osteoporosis and dementia, and may also experience issues such as kyphosis and osteoarthritis. Therefore, it is necessary to continuously monitor the physical health of the elderly at all times. However, the families of the elderly may find it difficult to make time to monitor the health status of the elderly due to work pressure.

Therefore, how to provide an analysis system that allows the elderly to easily operate and understand their own health status becomes a worthy topic for discussion.

### SUMMARY OF THE INVENTION

The primary objective of the present invention is to provide a health analysis system that analyzes health status according to the human posture.

To achieve the aforementioned objective, a health analysis system of the present invention is used for analyzing a health status of a user, and the health analysis system includes a camera, a posture recognition module, a posture analysis module and a health analysis module. The camera is used for capturing a user image of the user. The posture recognition module is electrically connected to the camera, and used for identifying a plurality of human skeleton points in the user image. The posture analysis module is electrically connected to the posture recognition module, and used for analyzing a human posture according to the plurality of human skeleton points. The health analysis module is electrically connected to the posture analysis module. The health analysis module further includes a skeleton analysis unit, and the skeleton analysis unit is used for analyzing a health status according to the human posture.

According to one embodiment of the present invention, the health analysis system further includes an infrasound module, and the infrasound module is electrically connected to the health analysis module.

According to one embodiment of the present invention, wherein the infrasound module further includes an infrasound player and an infrasound sensor; the infrasound player is used for playing an infrasound towards the user, and a body of the user which is in contact with the infrasound produces a rebound infrasound; the infrasound sensor is used for receiving the rebound infrasound.

According to one embodiment of the present invention, wherein the health analysis module further includes a physiological system analysis unit, and the physiological system analysis unit is used for analyzing the health status according to the rebound infrasound.

According to one embodiment of the present invention, the health analysis system further includes an electromagnetic induction module, wherein the electromagnetic induction module is electrically connected to the health analysis module, and the electromagnetic induction module is used for detecting an action and a temperature of the user via a far infrared.

According to one embodiment of the present invention, wherein the health analysis module further includes a lifestyle analysis unit, and the lifestyle analysis unit is used for analyzing the health status according to the action and the temperature.

According to one embodiment of the present invention, wherein the health analysis module further includes a light wavelength analysis unit, and the light wavelength analysis unit is used for analyzing the health status according to a skin color of the user image.

According to one embodiment of the present invention, the health analysis system further includes an indicator module, wherein the indicator module is electrically connected to the health analysis module, and the indicator module is used for providing an action instruction to the user, allowing the user to take a feedback action according to the action instruction.

According to one embodiment of the present invention, wherein the health analysis module further includes a feedback motion analysis unit, and the feedback motion analysis unit is used for analyzing the health status according to the feedback action.

According to one embodiment of the present invention, wherein the plurality of human skeleton points further includes a skull point, a left scapular point, a right scapular point, a left humerus point, a right humerus point, a left hip point and a right hip point.

### BRIEF DESCRIPTION OF THE DRAWINGS

FIG. 1 is a system structure diagram of the health analysis system and the mobile phone of one embodiment of the present invention.
FIG. 2 is a schematic diagram of the health analysis system of one embodiment of the present invention.
FIG. 3 is a schematic diagram of the plurality of human skeleton points in the user image of one embodiment of the present invention.
FIG. 4 is another schematic diagram of the plurality of human skeleton points in the user image of one embodiment of the present invention.

### DETAILED DESCRIPTION OF THE PREFERRED EMBODIMENT

To better understand the technical content of the present invention, a preferred specific embodiment is described as follows.

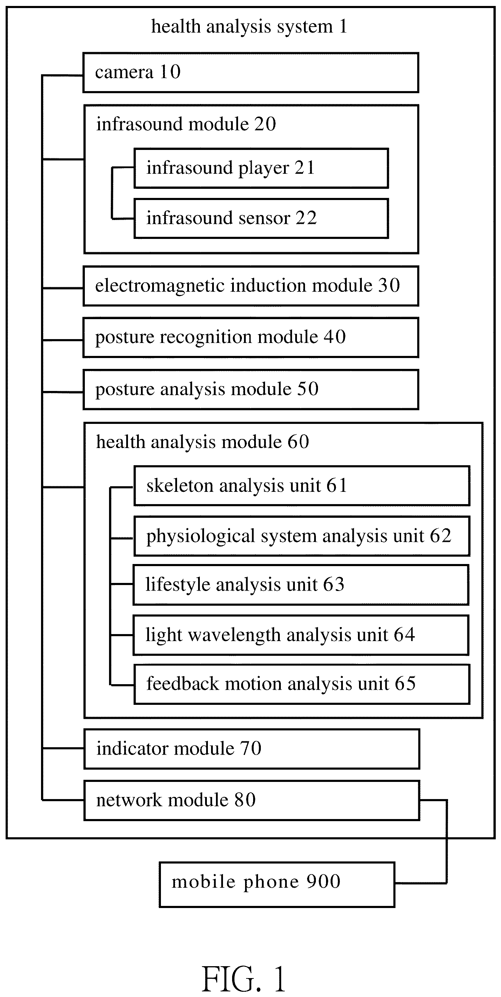

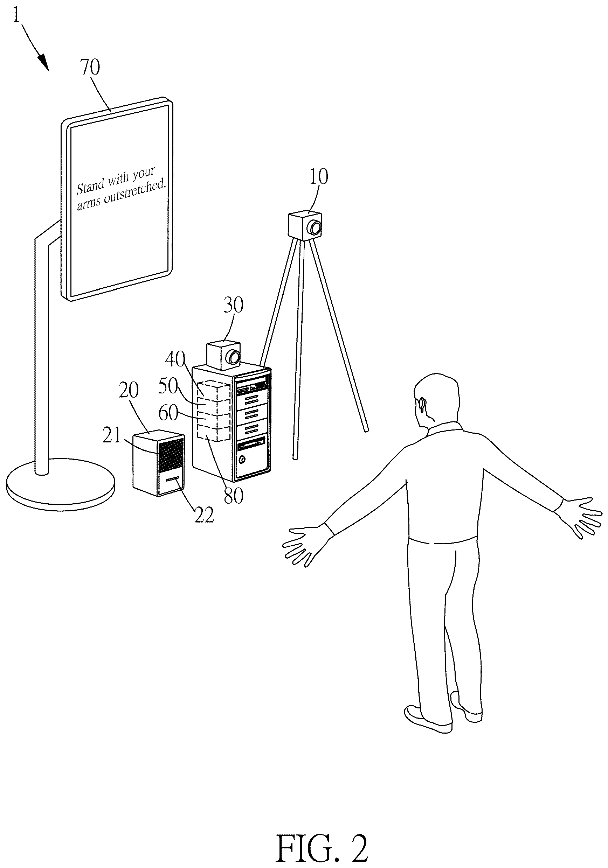

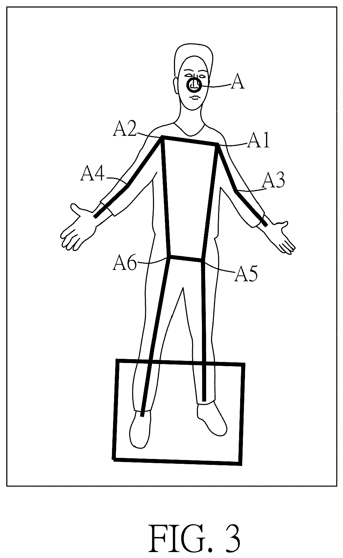

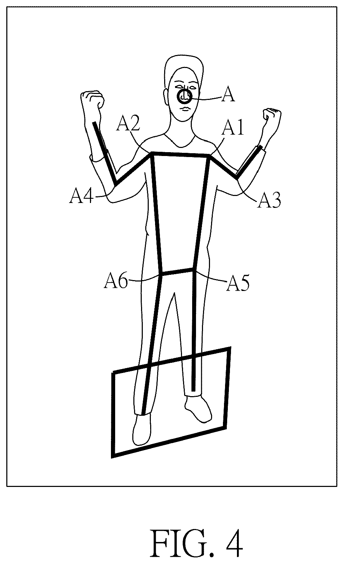

Referring to FIG. 1 to FIG. 4 for one embodiment of the health analysis system of the present invention. FIG. 1 is a system structure diagram of the health analysis system and the mobile phone of one embodiment of the present invention. FIG. 2 is a schematic diagram of the health analysis system of one embodiment of the present invention. FIG. 3 is a schematic diagram of the plurality of human skeleton points in the user image of one embodiment of the present invention. FIG. 4 is another schematic diagram of the plurality of human skeleton points in the user image of one embodiment of the present invention.

As shown in FIG. 1 and FIG. 2, in one embodiment of the present invention, the health analysis system can analyze a user's human health status based on the information presented by the user's body and present the human health status on a mobile phone 900. The health analysis system 1 includes a camera 10, an infrasound module 20, an electromagnetic induction module 30, a posture recognition module 40, a posture analysis module 50, a health analysis module 60, an indicator module 70 and a network module 80.

In one embodiment of the present invention, the camera 10 is used to capture a user image of the user, the user image is provided to other modules for analyzing the health status based on the user image. The infrasound module 20 is electrically connected to the health analysis module 60. The infrasound module 20 includes an infrasound player 21 and an infrasound sensor 22; the infrasound player 21, for example, is a speaker used to play infrasound (which is the sound waves with a frequency below 20Hz) to the user, causing the user's body which is contacted to the infrasound to resonate and produce a rebound infrasound. Specifically, the heart and lungs will resonate and produce rebound infrasound with specific frequencies (for example, the heart resonance frequency is 5Hz); the infrasound sensor 22, for example, is a microphone used to receive the rebound infrasound. However, the design of the infrasound module 20 is not limited to playing and receiving infrasound; it can also be used to play and receive sound waves with frequencies above 20Hz. Additionally, the sound waves that the infrasound module 20 can play and sense are not limited to infrasound; for example, the infrasound module 20 can also be designed to include a millimeter wave radar detector, which can play and detect millimeter waves (which can resonate with the human body's breathing and heartbeat) to analyze the target human body's breathing and heart rate in real-time.

In one embodiment of the present invention, the electromagnetic induction module 30 is an infrared sensor, which is electrically connected to the health analysis module 60. The electromagnetic induction module 30 is used to emit far infrared and detect the action and temperature of the user through the far infrared.

In one embodiment of the present invention, the posture recognition module 40 is a software or chip with the function of human image and corresponding skeletal structure analysis. The posture recognition module 40 is electrically connected to the camera 10 and used to identify the plurality of human skeleton points A~A6 in the user image captured by the camera 10. As shown in FIG. 3 and FIG. 4, the plurality of human skeleton points include a skull point A, a left scapular point A1, a right scapular point A2, a left humerus point A3, a right humerus point A4, a left hip point A5, and a right hip point A6. However, the human skeleton points are not limited to the above, and human skeleton points can be designed into other skeletal joints according to the needs, such as knee skeleton points, ankle skeleton points. Alternatively, the human structures that the posture recognition module 40 can identify are not limited to skeletal structures, and it can also be designed to identify human eyes, mouths, facial shapes, etc.

As shown in FIG. 1 and FIG. 2, in one embodiment of the present invention, the posture analysis module 50 is a software or chip with human image and corresponding human posture analysis functions, electrically connected to the posture recognition module 40, and is used to analyze a human posture based on the plurality of human skeleton points A~A6 identified by the posture recognition module 40. For example, a human posture can be analyzed as standing straight, squatting, sitting, hunching, pelvic forward tilt, hand raising, foot lifting, eye rotation, mouth shape change, tongue change, or other human actions based on the plurality of human skeleton points A~A6 and the user image. However, the posture analysis module 50 is not limited to the above-mentioned postures and can also analyze other human postures, such as facial expressions, eye opening and closing, eye ball rotation, mouth movements, tongue movements, hand movements, etc. Alternatively, the posture analysis module 50 can analyze the correlation between clear enunciation, speech, singing, etc. and mental, brain, and limb function.

In one embodiment of the present invention, the indicator module 70 is a mobile phone, tablet, or smart TV display, which is electrically connected to the health analysis module 60. The indicator module 70 is used to present a screen and can also provide an action instruction to the user, allowing the user to watch the screen and take a feedback action based on the action instruction. The action instruction, for example, uses text or images to instruct the user to perform specific actions (e.g., stand up, half-squat, take a deep breath, raise your hand, etc.). The network module 80, for example, is a network card, which is electrically connected to the health analysis module 60 and the mobile phone 900. The network module 80 is used to send the contents analyzed by the health analysis module 60 to the mobile phone 900 for the user to watch.

In one embodiment of the present invention, the health analysis module 60 is a software or chip with health analysis functions, which is electrically connected to the posture analysis module 50 and can analyze the target's breathing, heart rate, blood pressure, body temperature, skin color, and combine human posture and body movement collection reports to analyze the health status in real-time through the health analysis functions. The posture recognition module 40, posture analysis module 50, health analysis module 60, and network module 80 are all installed within a computer host, and the camera 10, infrasound module 20, electromagnetic induction module 30, and indicator module 70 are electrically connected to the posture recognition module 40, posture analysis module 50, and health analysis module 60 via wireless connection; however, the connection method is not limited to wireless and can be connected via cables as needed.

The health analysis module 60 further includes a skeleton analysis unit 61, a physiological system analysis unit 62, a lifestyle analysis unit 63, a light wavelength analysis unit 64, and a feedback motion analysis unit 65. The skeleton analysis unit 61 is used to analyze the health status based on human posture. The physiological system analysis unit 62 is used to analyze the health status based on rebound infrasound. The lifestyle analysis unit 63 is used to analyze the health status based on actions and body temperature, such as heart rate analysis. The light wavelength analysis unit 64 is used to analyze the health status based on the skin color in the user image, for example, by utilizing optical coherence tomography, which can accurately measure microscale structures within the eye. For instance, the concentration of advanced glycation end products (AGEs) in the body of diabetes patients will differ from that of healthy individuals. If there is a risk of disease, AGEs will abnormally accumulate over time within the eye. Therefore, by using optical coherence tomography, blue light that is invisible to the naked eye can be directed into the eye, allowing green light to enter the skin. The green fluorescence reflected by AGEs and the scattered blue light can then be detected, enabling the determination of the health status of the subject based on the reflected light and combining it with machine learning. The feedback motion analysis unit 65 is used to analyze the health status based on feedback actions.

As shown in FIG. 2 to FIG. 4, when a user wants to use the health analysis system 1 of this embodiment to view their own health status, the user can stand in front of the camera 10 and allow the camera 10 to take a user image. The user image will be sent to the posture recognition module 40 and the health analysis module 60. The posture recognition module 40 will identify the plurality of human skeleton points A~A6 in the user image taken by the camera 10 and send the user image and the plurality of human skeleton points A~A6 to the posture analysis module 50 and the indicator module 70. The posture analysis module 50 will analyze a human posture based on the plurality of human skeleton points A~A6 identified by the posture recognition module 40. For example, if the positions of the left hip point A5 and the right hip point A6 are close to the ground in the user image, it can be analyzed that the user is squatting. If the left scapular point A1, the right scapular point A2, the left humerus point A3, and the right humerus point A4 appear in a similar W-shaped line shape (as shown in FIG. 4), it can be analyzed that the user should raise their hands. Therefore, various human postures can be analyzed by combining the plurality of human skeleton points A~A6 with the user image. The human posture analyzed by the posture analysis module 50 will be sent to the skeleton analysis unit 61 of the health analysis module 60, and the skeleton analysis unit 61 will analyze whether the human posture is good or needs improvement and provide a score. The indicator module 70 will display the score from the skeleton analysis unit 61 and the user image, along with the plurality of human skeleton points A~A6, on the screen for the user to view and understand their own health status.

Additionally, the light wavelength analysis unit 64 of the health analysis module 60 analyzes the health status based on the skin color in the user image, such as darkening of the user's facial skin indicating kidney issues and pale facial skin indicating anemia. However, the diseases corresponding to the skin color are not limited to the above examples, and can be changed according to detection needs.

Additionally, the infrasound player 21 of the infrasound module 20 will play infrasound, causing the body of the user who is in contact with the infrasound to resonate and produce a rebound infrasound. The microphone of the infrasound sensor 22 will receive the rebound infrasound. The rebound infrasound received will be sent to the physiological system analysis unit 62. The physiological system analysis unit 62 will analyze the health status based on the rebound infrasound, for example, the frequency of the rebound infrasound produced by heart resonance is 5Hz, therefore the physiological system analysis unit 62 will recognize 5Hz as the rebound infrasound corresponding to the heart, and based on the vibration intensity and frequency of the 5Hz rebound infrasound, it can determine the operational status of the heart, thereby analyzing the health status of the heart.

Additionally, the electromagnetic induction module 30 can emit far infrared, detect the user's actions and body temperature, and send the detected action and body temperature data to the lifestyle analysis unit 63. The lifestyle analysis unit 63 will analyze the health status based on the received action and body temperature data.

Additionally, the indicator module 70 can also provide action instructions to the user, such as using text or images to indicate that the user should take a feedback action (e.g., asking the user to raise their hands). Since the camera 10 will capture the user image and send it to the health analysis module 60, the feedback motion analysis unit 65 of the health analysis module 60 will analyze whether the user's feedback action is correct based on the content of the user image and will analyze the health status based on the feedback action. For example, if the user correctly raises their hands according to the action instruction, it can be analyzed that the user's intelligence and physical ability are normal; if the user makes an incorrect motion, it can be analyzed that the user may have symptoms of dementia or reduced physical ability. In addition to checking the health status, the indicator module 70 can also provide a series of action instructions for the user to follow to exercise their body, thereby allowing the user to easily stay healthy by exercising at home. However, the usage of the indicator module 70 is not limited to the above, and the indicator module 70 can also be designed to work in conjunction with the infrasound module 20, for example, the indicator module 70 can use images to present a person making movements and speaking, and the indicator module 70 can also use text to instruct the user to follow the person in the image, making the same movements and speaking the same words. The microphone of the infrasound sensor 22 of the infrasound module 20 will receive the user's voice, and the infrasound module 20 will send the received voice to the health analysis module 60. The health analysis module 60 will analyze whether the user is correctly issuing voices based on the received voice. The camera 10 will capture the user's movements, and the health analysis module 60 will analyze whether the user is correctly following the indicator module 70's movements based on the images captured by the camera 10. In this way, it can be determined whether the user's physical and vocal coordination abilities are normal.

When the health analysis module 60 completes the analysis of the health status, the data of the health status will be transmitted to the network module 80. The network module 80 will send the contents analyzed by the health analysis module 60 to the mobile phone 900 for the user to view and record their health data. The mobile phone 900 can also be installed with software having AI question and answer functions, allowing the user to transmit their health data to AI for more detailed health explanations. Additionally, AI can also evaluate, predict, and simulate the user's actions in real-time based on images or photos taken by the camera 10, to determine whether the user's physical ability is normal.

Via the design of the health analysis system 1 of the present invention, the health analysis system 1 can analyze the health status of a user based on the information presented by the user's body. The health analysis system 1 can also provide a series of action instructions for the user to follow to exercise the body. Additionally, the health analysis system 1 can be easily assembled, conveniently stored, and allows for easy user access. Besides placing it at home, it is also suitable for placing in community areas frequently visited by the elderly (such as activity centers, clinics), allowing the elderly to check their health status nearby in the community.

It should be noted that the above is merely an embodiment and not limited to that embodiment. For example, if it does not deviate from the basic framework of the present invention, it should be within the scope of the claims of the present patent, and the scope of the patent application should be referred to.

## Claims

1. A health analysis system (1), used for analyzing a health status of a user, the health analysis system (1) comprising:
a camera (10), used for capturing a user image of the user;
a posture recognition module (40), electrically connected to the camera (10), and used for identifying a plurality of human skeleton points (A, A1, A2, A3, A4, A5, A6) in the user image;
a posture analysis module (50), electrically connected to the posture recognition module (40), and used for analyzing a human posture according to the plurality of human skeleton points (A, A1, A2, A3, A4, A5, A6); and
a health analysis module (60), electrically connected to the posture analysis module (50), wherein the health analysis module (60) further comprises a skeleton analysis unit (61), the skeleton analysis unit (61) is used for analyzing the health status according to the human posture.

2. The health analysis system (1) as claimed in Claim 1, further comprising an infrasound module (20), wherein the infrasound module (20) is electrically connected to the health analysis module (60).

3. The health analysis system (1) as claimed in Claim 2, wherein the infrasound module (20) further comprises an infrasound player (21) and an infrasound sensor (22); the infrasound player (21) is used for playing an infrasound towards the user, and a body of the user which is in contact with the infrasound produces a rebound infrasound; the infrasound sensor is used for receiving the rebound infrasound.

4. The health analysis system (1) as claimed in Claim 3, wherein the health analysis module (60) further comprises a physiological system analysis unit (62), and the physiological system analysis unit (62) is used for analyzing the health status according to the rebound infrasound.

5. The health analysis system (1) as claimed in Claim 4, further comprising an electromagnetic induction module (30), wherein the electromagnetic induction module (30) is electrically connected to the health analysis module (60), and the electromagnetic induction module (30) is used for detecting an action and a temperature of the user via a far infrared.

6. The health analysis system (1) as claimed in Claim 5, wherein the health analysis module (60) further comprises a lifestyle analysis unit (63), and the lifestyle analysis unit (63) is used for analyzing the health status according to the action and the temperature.

7. The health analysis system (1) as claimed in Claim 6, wherein the health analysis module (60) further comprises a light wavelength analysis unit (64), and the light wavelength analysis unit (64) is used for analyzing the health status according to a skin color of the user image.

8. The health analysis system (1) as claimed in Claim 7, further comprising an indicator module (70), wherein the indicator module (70) is electrically connected to the health analysis module (60), and the indicator module (70) is used for providing an action instruction to the user, allowing the user to take a feedback action according to the action instruction.

9. The health analysis system (1) as claimed in Claim 8, wherein the health analysis module (60) further comprises a feedback motion analysis unit (65), and the feedback motion analysis unit (65) is used for analyzing the health status according to the feedback action.

10. The health analysis system (1) as claimed in Claim 9, wherein the plurality of human skeleton points (A, A1, A2, A3, A4, A5, A6) further comprises a skull point, a left scapular point, a right scapular point, a left humerus point, a right humerus point, a left hip point, and a right hip point.
